# EUROPÄISCHE PATENTSCHRIFT

(11) **EP 0 906 572 B1**
(45) Veröffentlichungstag und Bekanntmachung des Hinweises auf die Patenterteilung: **03.04.2002**
(21) Anmeldenummer: 97927032.9
(22) Anmeldetag: 23.05.1997
(51) Int. Cl.: G01N 33/543, G01N 33/53, G01N 33/50

(54) **MASKIERUNG DER HINTERGRUNDFLUORESZENZ UND -LUMINESZENZ BEI DER OPTISCHEN ANALYSE BIOLOGISCH MEDIZINISCHER ASSAYS**
MASKING BACKGROUND FLUORESCENCE AND LUMINESCENCE IN OPTICAL ANALYSIS OF BIOMEDICAL ASSAYS
MASQUAGE DE LA FLUORESCENCE ET DE LA LUMINESCENCE D'ARRIERE PLAN LORS DE L'ANALYSE OPTIQUE D'ESSAIS BIOMEDICAUX

(30) Priorität: 28.05.1996 DE 19621312
(43) Veröffentlichungstag der Anmeldung: 07.04.1999
(73) Patentinhaber: BAYER AG, 51368 Leverkusen (DE)
(72) Erfinder: KRAHN, Thomas, D-58135 Hagen (DE); PAFFHAUSEN, Wolfgang, D-51381 Leverkusen (DE); SCHADE, Andreas, D-45277 Essen (DE); BECHEM, Martin, D-42111 Wuppertal (DE); SCHMIDT, Delf, D-42113 Wuppertal (DE)
(86) Internationale Anmeldenummer: EP9702662
(87) Internationale Veröffentlichungsnummer: WO9745739

(56) Entgegenhaltungen:
- WO-A-94/02642

## Beschreibung

Die Erfindung geht aus von einem Verfahren zur quantitativen optischen Analyse von fluoreszenzmarkierten biologischen Zellen, die mit einer Fluoreszenzfarbstofflösung in Kontakt stehen oder von lumineszenten Zellen, die in Form einer zusammenhängenden Zellschicht auf einem transparenten Träger am Boden eines Reaktionsgefäßes aufgebracht sind, oder auch von Fluoreszenz- oder Lumineszenz-markierten Reaktionspartnern in einer Lösung, in der ein fluoreszierender oder lumineszenter Ligand gelöst ist, wobei die Lösung mit einer für diesen Ligand spezifischen, am transparenten Träger am Boden des Reaktionsgefäß befindlichen Rezeptorschicht in Kontakt steht, deren für die Rezeptor-Liganden Bindung charakteristische Fluoreszenz- oder Lumineszenzstrahlung durch den transparenten Boden hindurch erfaßt und ausgewertet wird.

Aus WO 94/02642 ist es bekannt fluoreszenz-markierte target-spezifische Moleküle zum Nachweis des entsprechenden Targets an der Zelle zu verwenden. Dazu wird die Zelle mit einer Lösung enthaltend die fluoreszenz-markierten target-spezifischen Moleküle in Kontakt gebracht. Nach einer entsprechenden Inkubationszeit wird die Lösung entfernt und die Zelle dem Anregungslicht ausgesetzt und anschließend die Fluoreszenzemission gemessen. Dabei tritt das Problem auf, daß einige der fluoreszenz-markierten target-spezifischen Moleküle auch nicht-target-spezifische Bindungen eingehen und daß eine Lichtemission von zellulären Molekülen auftritt. Um diese unerwünschte Hintergrund-Emission zu unterdrücken wird zu der Lösung in der sich die Zelle befindet eine Verbindung gegeben, die diese Hintergrund-Emission einerseits absorbiert und andererseits die nicht-target-spezifischen Bindungsstellen besetzt.

Ein Problem bei der Fluoreszenzmessung in biologisch medizinischen Assays besteht häufig darin, daß die mit der biologischen Zellaktion korrelierten Fluoreszenzänderungen klein sind gegenüber der unspezifischen Hintergrundfluoreszenz aus dem Überstand der Fluoreszenzfarbstofflösung mit der die Zellen in Kontakt stehen. Dadurch wird das Auflösungsvermögen stark eingeschränkt. Herkömmliche kommerzielle Meßsysteme (Fluoreszenzreader, Fa. Dynatech bzw. SLT) können das Problem nicht lösen, weil durch ihre optischen Meßanordnung (Anregung von 'oben' durch die fluoreszente Flüssigkeitssäule des Überstands) das Signal im Vergleich zum Hintergrund kaum detektiert werden kann. Geräte neuerer Konstruktion (Fa. Labsystems), die die Zellen von der Rückseite durch den transparenten Träger des Reaktionsgefäßes beleuchten, haben zwar den Vorteil, daß bei Eintritt des Anregungslichts die Zellen zur Fluoreszenz angeregt werden. Da das Anregungslicht aber weiter in den ebenfalls fluoreszenten Überstand eintritt, läßt es sich nicht vermeiden, daß das unspezifische Hintergrundsignal das Zellsignal verfälscht. Selbst sehr aufwendige Meßsysteme (Fa. NovelTech, FLIPR: Flurescence Imaging Plate Reader) können mit einer speziellen Laser-Beleuchtungsgeometrie (Anregung unter ca. 45°) diese Hintergrundfluoreseszenz nur vermindern. Grund für das Scheitern aller Problemlösungsversuche über die Meßgeometrie ist der Umstand, daß hierüber die eigentliche Ursache für die Hintergrundfluoreszenz nicht entscheidend beeinflußt werden kann.

Bei den bisher durchgeführten Rezeptor-Bindungstudien mit Fluoreszenz- oder Lumineszenz-markierten Liganden muß der jeweils markierte und nicht gebundene Anteil durch waschähnliche Vorgänge entfernt werden. Viele Beschichtungen sind jedoch empfindlich für diese Waschschritte. Außerdem ist das Entfemen des ungebundenen Liganden mit einem beträchtlichen Aufwand verbunden. Die direkte Messung der Rezeptor-Ligand Assoziation bzw. Dissoziation ist bei diesem Verfahren nicht möglich.

Der Erfindung liegt die Aufgabe zugrunde, die Empfindlichkeit der optischen Analyse von fluoreszenzmarkierten oder lumineszenten Zellen in einem zellulären Assay zu verbessern, um z. B. möglichst geringe Membranpotentialänderungen auf der Basis von Fluoreszenzänderungen potentialsensitiver Farbstoffe messen zu können. Dabei soll die Empfindlichkeit des Meßsystems so hoch sein, daß sich Potentialänderungen unter 5 mV mindestens qualitativ nachweisen lassen. Im Falle von lumineszenten Zellen soll eine Steigerung in der Detektion des Lumineszenzsignals erreicht werden. Außerdem soll die Methode für ein Screening mit hohem Probendurchsatz geeignet sein.

Weiterhin liegt der Erfindung die Aufgabe zugrunde, Rezeptor-Bindungsstudien auf der Basis von Fluoreszenz- bzw. Lumineszenz-markierten Liganden bzw. Rezeptoren zu vereinfachen und eine kontinuierliche Messung der Rezeptor-Bindungsinteraktion (Kinetik) zu ermöglichen. Durch die Reduzierung der erforderlichen Verfahrensschritte soll diese Methode insbesondere für ein Screening mit hohem Durchsatz und für diagnostische Anwendungen geeignet sein.

Die geforderte hohe Auflösung bei geringen Membranpotentialänderungen konnte erst erzielt werden, nachdem die Ursache für die störende Überlagerung der unspezifischen Hintergrund- und der spezifischen Fluoreszenz der Zellen beseitigt werden konnte. Das hierzu entwickelte erfindungsgemäße Verfahren beruht auf der grundsätzlich neuen Idee, die Anregungsenergie und die nicht von dem biologischen Objekt stammende Fluoreszenz zu maskieren. Hierzu wird neben dem Fluoreszenzfarbstoff ein weiterer Farbstoff hinzugefügt, der das Anregungslicht des Fluoreszenzfarbstoffs und/oder dessen Emissionslicht vollkommen absorbiert, ohne die Fluoreszenz der Zellen zu beeinflussen. Durch diese Absorption wird erreicht, daß das unspezifische Hintergrundsignal maskiert und das Zell-Nutzsignal mit einer bisher nicht möglichen Auflösung detektiert werden kann.

Eine im Rahmen der Erfindung liegende Alternativlösung besteht darin, daß auf die Zellschicht eine für die Lösung durchlässige Trennschicht aufgebracht wird, die das Anregungslicht für den Fluoreszenzfarbstoff und/oder sein Emissionslicht absorbiert und/oder reflektiert, ohne die Zelleigenschaften negativ zu beeinflussen. Dabei wird die Dicke der Trennschicht so gewählt, daß im Lösungsansatz mit dem Fluoreszenzfarbstoff aber ohne die Zellen keine Fluoreszenz mehr nachweisbar ist.

Eine weitere Variante der Erfindung besteht darin, daß die Methode der erfindungsgemäßen Trennschicht auch zur Empfindlichkeitssteigerung bei der quantitativen optischen Analyse von lumineszenten (selbstleuchtenden) biologischen Zellen verwendet wird, die in Form einer zusammenhängenden Zellschicht auf einem transparenten Träger aufgebracht sind. Zu diesem Zweck werden die optischen Eigenschaften, der für die Lösung durchlässigen Trennschicht so gewählt, daß sie das Lumineszenzlicht möglichst stark reflektiert, ohne die Zelleigenschaften negativ zu beeinflussen. Auf diese Weise kann die Lumineszenzintensität und damit der Meßeffekt beträchtlich erhöht werden.

Das erfindungsgemäße Verfahren kann in ganz analoger Weise zur quantitativen optischen Analyse von Fluoreszenz- oder Lumineszenz-markierten Reaktionspartnern in einem mit einer Lösung gefüllten Reaktionsgefäß herangezogen werden, wobei der fluoreszierende oder lumineszente Ligand in gelöster Form vorliegt und die Lösung mit einer für diesen Ligand spezifischen, auf einem transparenten Träger am Boden des Reaktionsgefäß aufgebrachten oder sich darauf absetzenden Rezeptorschicht in Kontakt steht, deren für die Rezeptor-Liganden Bindung charakteristische Fluoreszenz- oder Lumineszenzstrahlung durch den transparenten Boden hindurch erfaßt und ausgewertet wird. In diesem Fall beruht die erfindungsgemäße Lösung der oben beschriebenen Aufgabe darauf, daß der sich im Überstand, d.h. in Lösung befindliche freie Ligand und dessen unspezifische Fluoreszenz oder Lumineszenz durch einen zusätzlichen Farbstoff und/oder durch eine diffus absorbierende oder reflektierende Trennschicht maskiert wird und damit die Ursache für die störende Überlagerung der unspezifischen Hintergrund- und der spezifischen Fluoreszenz der Liganden in der Lösung beseitigt wird. Da auf diese Weise der nicht gebundene Ligand maskiert wird, stellt die gemessene Fluoreszenz oder Lumineszenz ein direktes Maß für die Interaktion Ligand-Rezeptor dar. Sie kann bei diesem Verfahren zeitlich aufgelöst direkt gemessen werden.

Gegenstand der Erfindung ist also bei Rezeptorstudien in Analogie zu dem oben beschriebenen Verfahren eine Verfahrensvariante, bei der der Lösung ein Maskierungsfarbstoff hinzugefügt und/oder auf die Rezeptorschicht eine für die Lösung durchlässige Trennschicht aufgebracht wird, wobei die optischen Eigenschaften des Maskierungsfarbstoffs und/oder der Trennschicht so gewählt werden, daß das Anregungslicht für den Fluoreszenzfarbstoff des in der Lösung vorhandenen Liganden und/oder sein Emissionslicht oder sein Lumineszenzlicht von der Lösung oder der Trennschicht absorbiert oder an der Trennschicht reflektiert wird. Dabei wird die Dicke der Trennschicht so gewählt, daß im Lösungsansatz mit dem Fluoreszenzfarbstoff, aber ohne die Rezeptorschicht, keine Fluoreszenz mehr nachweisbar ist.

Vorzugsweise besteht die Trennschicht aus polymeren Latexkügelchen (z.B. Polystyrol, Polyurethan, Butadien Acrylnitril). Die Latexkügelchen können dabei auch mit einem Maskierungsfarbstoff gefärbt sein, der in diesem Fall eine hinreichend große Polymeranfärbbarkeit aufweisen muß.

Bei dem zuerst erwähnten Verfahren soll sich der Maskierungsfarbstoff möglichst gut in der Lösung, die auch den Fluoreszenzfarbstoff in gelöster Form enthält, verteilen. Da das Lösungsmittel in der Regel Wasser ist, wird zweckmäßig ein Maskierungsfarbstoff eingesetzt, der eine gute Wasserlöslichkeit besitzt (>2g/ml) und keine zelltoxischen Nebeneffekte aufweist.

Gemäß einer Weiterentwicklung der Erfindung wird nach dem Austausch des einen Fluoreszenzfarbstoff enthaltenden Überstandes durch eine Fluoreszenzfarbstoff-freie Lösung ein weiterer Maskierungsfarbstoff zugegeben, welcher eine unspezifische Fluoreszenz an der Reaktionsgefäßwand unterdrückt.

Mit der Erfindung werden folgende Vorteile erzielt:
Das beschriebene neue Verfahren ist nicht an ein bestimmtes Meßsystem gebunden, sondern kann, weil es keine spezifisch technische Lösung darstellt, von vielen handelsüblichen Geräten benutzt werden. Hierzu zählen praktisch alle Fluoreszenzreader, die transparente Reaktionsgefäße z.B. Mikrotiterplatten von der Unterseite her beleuchten und auch messen können. Mit einem sehr geringen Aufwand (minimale Zusatzkosten nur für die speziellen Absorptionsfarbstoffe) wird es hierdurch erstmals möglich, in einen Auflösungsbereich z.B. bei der Messung von Potentialänderungen in Zellmembranen durch Messung der Fluoreszenzänderung potentialsensitiver Fluoreszenzfarbstoffe vorzudringen, der bisher unerreicht war. Erstmals wird es möglich, auch bei sehr kleinen Änderungen einen direkten Vergleich der Ergebnisse aus verschiedenen Reaktionsgefäßen (z.B. verschiedene Vertiefungen in einer Mikrotiterplatte) durchzuführen, so daß auf das aufwendige Verfahren der Bestimmung der relativen Änderung in einem Reaktionsgefäß verzichtet werden kann. Dadurch verringert sich die Anzahl der zu erfassenden Meßwerte z.B. für Kinetikmessungen. Der zeitliche Aufwand für ein Meßprogramm wird deutlich reduziert und die Möglichkeit geschaffen, durch eine simple Einzelmessung (z.B. Endpunktbestimmung) unter Verwendung des Bezugs auf einen getrennten Kontrollansatz gleiche Resultate zu erhalten. Die hierbei geforderte Uniformität des biologischen Ansatz (z.B. homogene Zellschicht) ist z.B. für Mikrotiterplatten im allgemeinen gegeben.

Überraschenderweise zeigte die Anwendung verschiedener wasserlöslicher Farbstoffe und auch deren Mischungen in den verschiedensten getesteten Zellen keine negative Auswirkung auf die Physiologie der Zellen (z.B. Reaktion der Zellen im Vergleich zu elektrophysiologischen Messungen wie Whole-cell-patch-clamp, bzw. Effekte der untersuchten Pharmaka). Auch der Einsatz von unlöslichen Farbpigmenten bzw. anorganischen feinverteilten Teilchen wurde erstaunlich gut von den biologischen Objekten toleriert.

Durch das beschriebene einfache Verfahren der Maskierung der Hintergrundfluoreszenz bei der quantitativen Fluoreszenzmessung in biologisch medizinischen Assays verbunden mit einer Steigerung der Empfindlichkeit z. B. beim Einsatz von potentialsensitiven Fluoreszenzfarbstoffen und die Adaptionsfähigkeit dieses Verfahrens z.B. auf Mikrotiterplatten als Reaktionsgefäße wird der Einsatz solcher Meßtechniken das High-Throughput-Screening wesentlich vereinfachen, zumal zur Realisierung der geschilderten Vorteile kein erhöhter technischer Aufwand notwendig ist, sondern vorhandene kommerzielle Meßgeräte dazu ausreichen.

Bei Rezeptor-Liganden Studien liegt der erfindungswesentliche Vorteil darin, daß es aufgrund der Maskierung der unspezifischen Fluoreszenz bzw. Lumineszenz nicht mehr erforderlich ist, den nicht gebundenen Anteil der Liganden zu entfernen. Dadurch werden die Testverfahren erheblich vereinfacht, Beschädigungen und Zerstörungen der empfindlichen Beschichtungen bzw. der biologischen Objekte wie z.B. Zellen, vermieden und die Empfindlichkeit und damit auch die Genauigkeit der Messung verbessert. Durch die Verwendung von Mikropartikeln kann die nutzbare Oberfläche zur Beschichtung von Fluoreszenz- oder Lumineszenz-markierten Liganden wesentlich vergrößert werden. Durch geeignete Maßnahmen, z.B. höhere spezifische Dichte bzw. die Verwendung von magnetisierbaren Partikeln. kann erreicht werden, daß sich die Mikropartikel am tranparenten Träger absetzen und anreichern. Auch in diesem Fall wird die Fluoreszenz bzw. Lumineszenz der nicht gebundenen, im Überstand befindlichen Liganden durch die Maskierung wirkungsvoll unterdrückt.

Da die Interaktion zwischen dem Liganden und dem Rezeptor nicht durch das Entfernen des ungebundenen Anteils unterbrochen weden muß, kann auf diese Weise sogar in einem einzelnen Reaktionsansatz eine kontinuierliche Messung der Interaktion zwischen Ligand und Rezeptor (Kinetik) erfolgen.

Im Folgenden wird die Erfindung an Hand von Ausführungsbeispielen und Zeichnungen näher erläutert: Es zeigen:
- Fig. 1: ein Reaktionsgefäß für einen Fluoreszenzassay nach dem Stand der Technik
- Fig. 2: die Unterdrückung der Hintergrundfluoreszenz bei einem Fluoreszenzassay mit einem Maskierungsfarbstoff im Überstand
- Fig. 3: die spektrale Anregung und Emission für einen Verteilungsfarbstoff und die spektrale Absorption des Maskierungsfarbstoffes
- Fig. 4: die ortsabhängige Zellfluoreszenz ohne Maskierungsfarbstoff
- Fig. 5: die ortsabhängige Zellfluoreszenz mit Maskierungsfarbstoff
- Fig. 6: die Unterdrückung der Hintergrundfluoreszenz bei einem Fluoreszenzassay mit Hilfe einer Trennschicht
- Fig. 7: die Verstärkung der Lumineszenz durch Rückreflexion an einer Trennschicht
- Fig. 8: die Wandfluoreszenz bei einem Fluoreszenzassay nach dem Stand der Technik

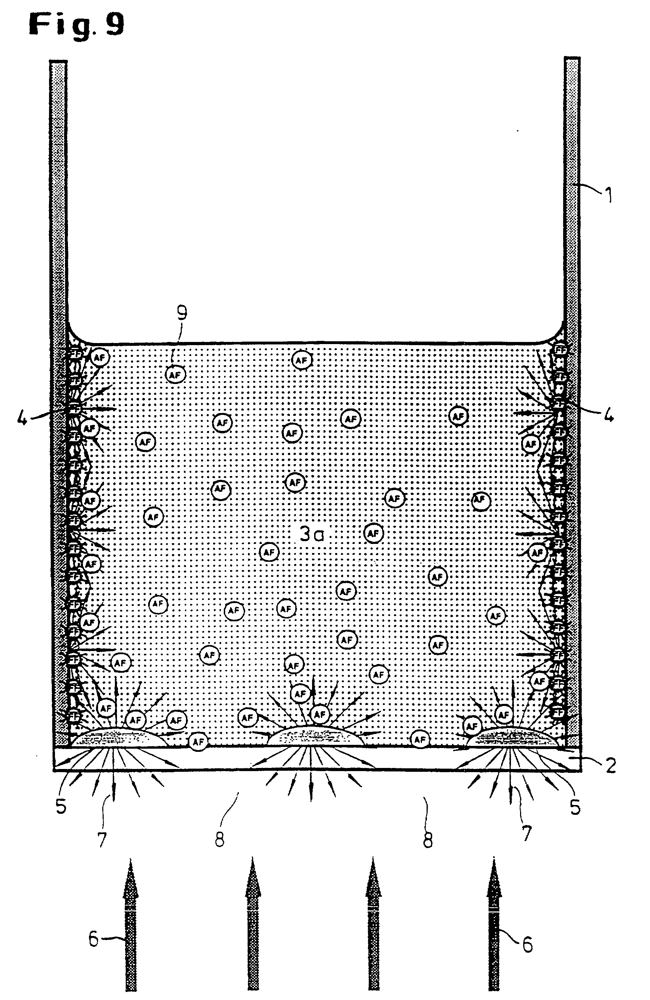

- Fig. 9: die Unterdrückung der Wandfluoreszenz bei einem Fluoreszenzassay mit Hilfe eines Maskierungsfarbstoffes und
- Fig. 10: ) die Unterdrückung der Hintergrund-fluoreszenz oder lumineszenz bei einem Fluoreszenz- oder Lumineszenzassay zur Untersuchung von Rezeptor-Ligandenbindungen mit Hilfe eines Maskierungsfarbstoffs im Überstand

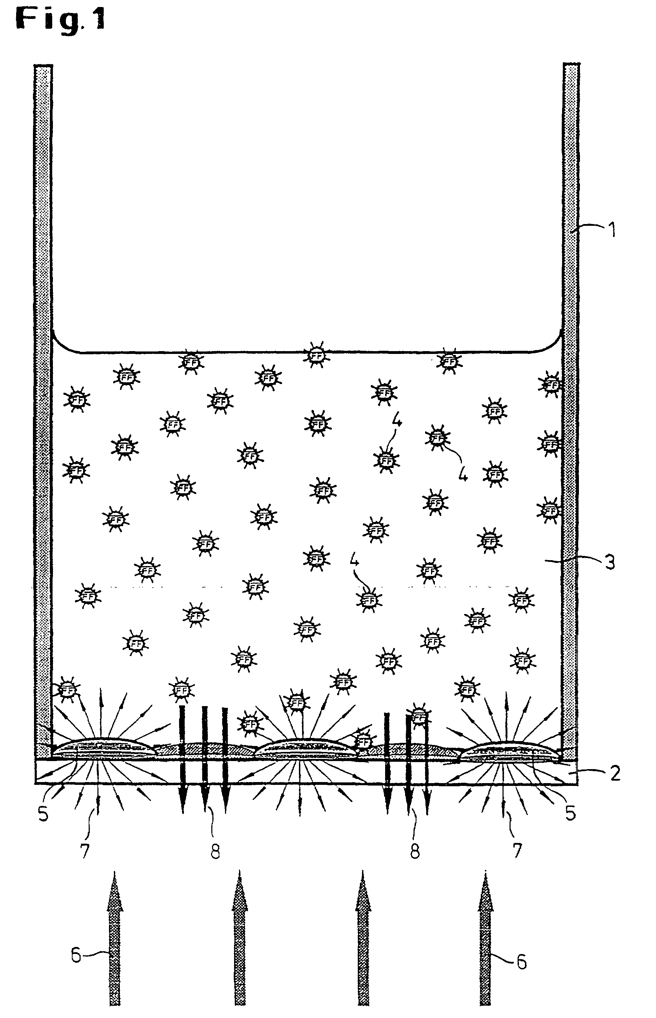

In Fig. 1 ist ein Reaktiongefäß 1 für ein Fluoreszenzassay mit einem transparenten Boden 2 dargestellt. In dem Reaktionsgefäß 1 befindet sich eine Fluoreszenzfarbstofflösung 3, in der die Fluoreszenzfarbstoffmoleküle 4 schematisch angedeutet sind. Die Lösung 3 wird auch als Überstand bezeichnet. Auf dem transparenten Boden 2 sind die zu untersuchenden biologischen Zellen auf einem transparenten Träger angeordnet. Durch den Boden 2 wird Licht (Anregungslicht) 6 eingestrahlt, um die Zellen 5 zur Fluoreszenz anzuregen. Dem von den Zellen 5 ausgestrahlten Fluoreszenzlicht 7 ist eine Hintergrundfluoreszenz-Strahlung 8 überlagert, die von den ebenfalls angeregten Fluoreszenzfarbstoffmolekülen 4 im Überstand 3 herrührt. Für die biologischanalytische Untersuchung und Auswertung der Zellen 5 ist aber nur das Fluoreszenzlicht 7 maßgebend. Da aber bei allen bekannten Fluoreszenz-Analysegeräten die Hintergrundfluoreszenz 8 mit erfaßt wird, gehen kleine Fluoreszenzunterschiede der Zellen 5 vor der starken Hintergrundfluoreszenz 8 unter, was zu einem deutlichen Empfindlichkeitsverlust führt.

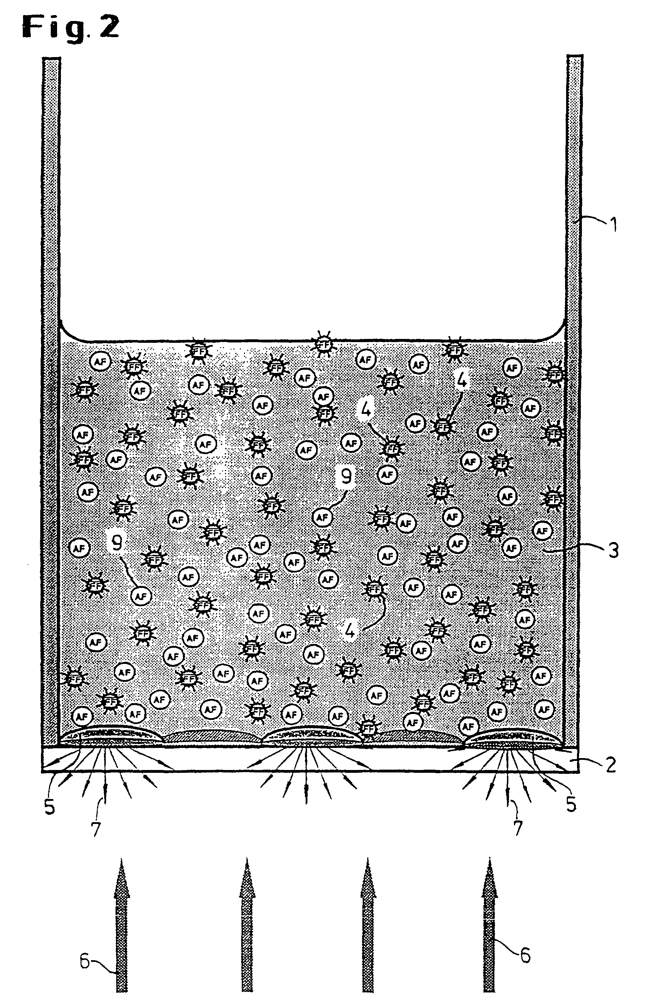

Dieser Nachteil kann durch das erfindungsgemäße Verfahren nach Fig. 2 dadurch vermieden werden, daß die Hintergrundfluoreszenz durch einen Maskierungsfarbstoff im Überstand 3 unterdrückt wird. Die in Fig. 1 vorhandene Hintergrundfluoreszenz 8 wird gem. Fig. 2 vollständig im Überstand absorbiert. Der zu dem Überstand 3 hinzugefügte Maskierungsfarbstoff (schematisch mit 9 bezeichnet) kann entweder in gelöster Form oder in fein verteilter disperser Phase (farbpigmentierte Systeme) vorliegen. Bevorzugt werden jedoch lösliche Farbstoffe eingesetzt, weil hier die Zugabe besonders einfach mit Hilfe einer Pipette erfolgen kann und weil im Gegensatz zu einem Pigment-System die physikalischen Einflüsse der Teilchengrößenverteilung und von Sedimentationsprozessen und Schichtdickenungleichmäßigkeiten nicht berücksichtigt werden müssen.

An die Eigenschaften eines derartigen Farbstoffs werden folgende Anforderungen gestellt:
- bei Verwendung eines löslichen Absorptionsfarbstoffes gute Wasserlöslichkeit für den Einsatz in biologischen Assays
- keine Membrangängigkeit des Farbstoffs, um eine Anfärbung der Zellen zu vermeiden
- hohe spezifische Absorption im Excitations- und/oder Emissions-Wellenlängenbereich des Fluoreszenzfarbstoffes
- keine toxischen Nebeneffekte (Vermeidung von Zellschädigungen)

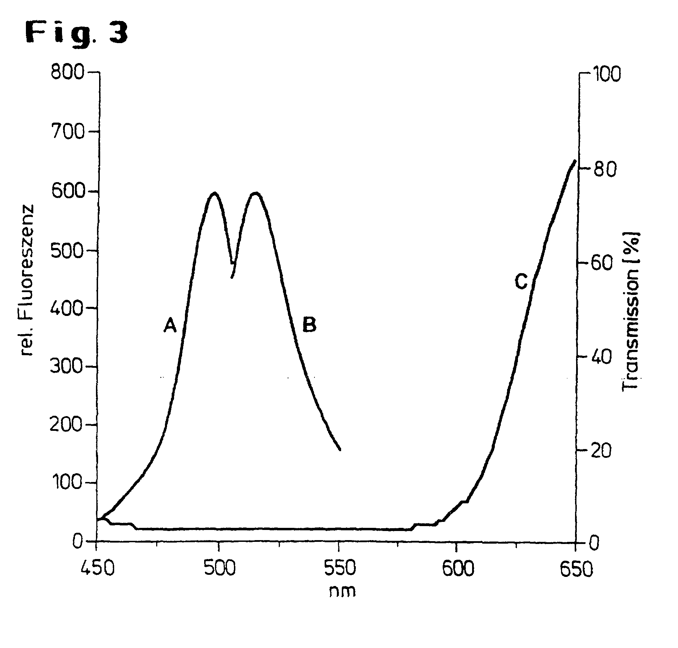

Als gute Wasserlöslichkeit wird eine Löslichkeit von >2 mg/ml angesehen. Die Zelltoxizität kann mit Hilfe bekannter Testverfahren (z.B. Zytotoxizitätstest) bestimmt werden. Fig. 3 zeigt die optischen (spektralen) Eigenschaften eines Fluoreszenz- und eines Maskierungsfarbstoffes in einem Diagramm. Die Kurve A zeigt die spektrale Verteilung des Anregungslichtes, die Kurve B die spektrale Verteilung des emittierten Fluoreszenzlichts für den handelsüblichen Verteilungsfluoreszenz-Farbstoff Bis-(1,3-dibutylbarbituricacid)trimethaneoxonol (Dibac₄(3)) und die Kurve C die spektrale Transmission (Absorptionsspektrum) des verwendeten Maskierungsfarbstoffs (Brilliant Black BN, C.I. 28440, Food Black 1, z.B. Sigma B-8384). Man erkennt, daß der Maskierungsfarbstoff im Wellenlängenbereich der Anregung und Emission des Fluoreszenzfarbstoffs fast vollständig absorbiert.

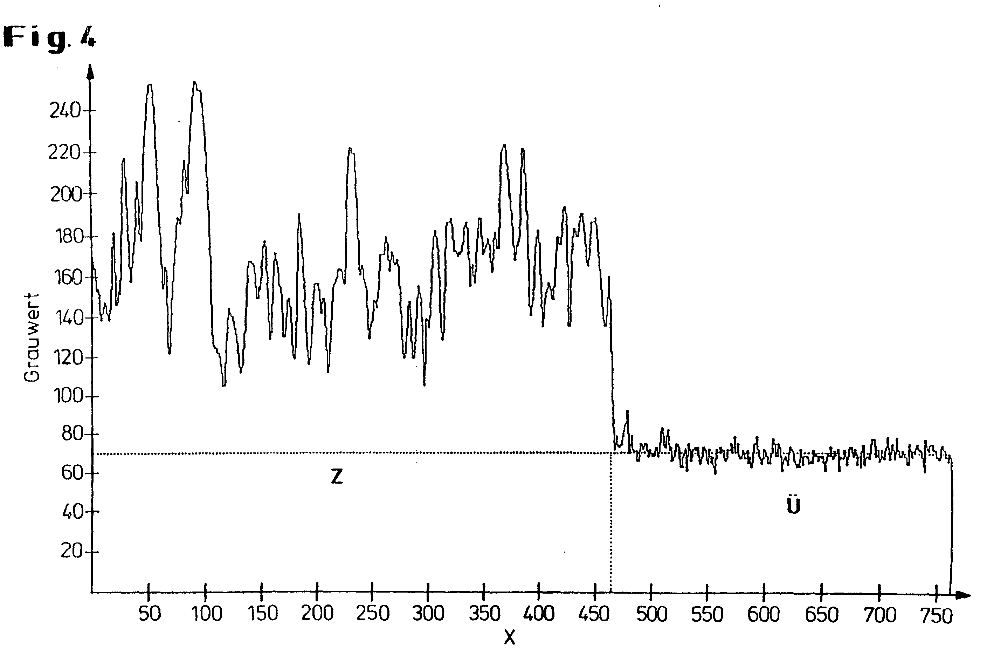

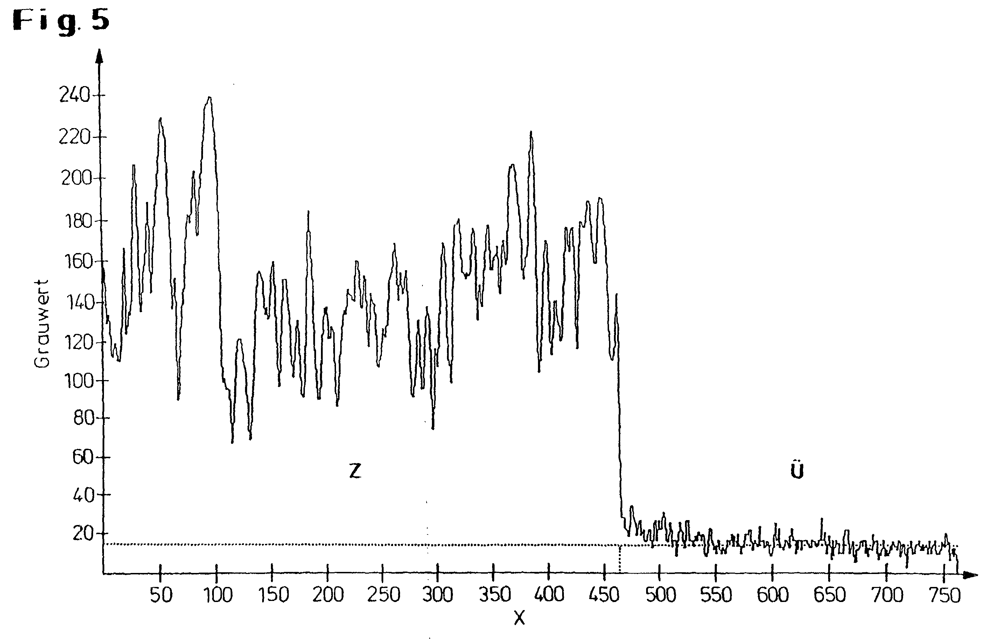

Die Kontrastverbesserung bzw. Empfindlichkeitssteigerung läßt sich noch besser an Hand der Figuren 4 und 5 verstehen. Zur Demonstration der Wirkung des Maskierungsfarbstoffs auf die unspezifische Hintergrundfluoreszenz wurden zwei Videoaufnahmen von dem gleichen Bildausschnitt vor und nach Hinzufügen von 100 mg/ml des löslichen Maskierungsfarbstoffes Brilliantschwarz in Anwesenheit des potentialsensitiven Fluoreszenzfarbstoffes Dibac₄(3) (5 mM) gemacht. Beide Male wurden dieselbe Videozeile bildanalytisch ausgewertet und die beiden Fluoreszenzintensitätsprofile über die identischen Ausschnitte im Reaktionsgefäß dargestellt. Der Bereich Z entspricht dabei dem Bereich, in dem sich die Zellschicht, d.h. die biologische Probe befindet, während rechts davon in der Zone Ü zum größten Teil das vom Überstand herkommende Fluoreszenzsignal gemessen wird. Der Meßbereich des Aufnahmesystems (8 bit) liegt zwischen 0 (schwarz) und 255 (weiß). Für die unmaskierte Aufnahme ergibt sich ein Kontrastverhältnis von ca. 1:3,6 (Intensitätsverhältnis der dunkelsten und hellsten Bildanteile) und im Falle der maskierten Aufnahme ein Kontrastverhältnis von ca. 1:14,4. Das entspricht einer Kontraststeigerung um den Faktor 4.

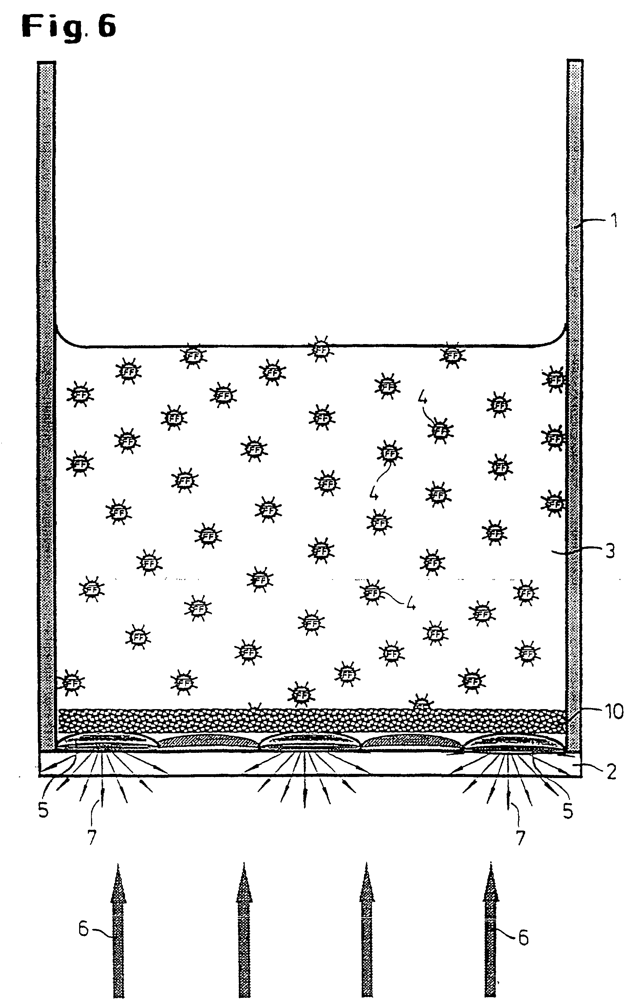

Eine alternative Möglichkeit, das Verhältnis von Nutz- zu Hintergrundsignal zu verbessern, besteht gemäß Fig. 6 in einer Überlagerung der Zellschicht mit einer feinteiligen optischen Trennschicht 10. Die Trennschicht 10 besteht zweckmäßig aus einem feinteiligen anorganischen Weißpigment, wie z.B. TiO₂ oder Al₂O₃. Hierdurch wird nicht nur die Hintergrundfluoreszenzstrahlung aus dem Überstand 3 abgeschirmt, sondern auch die meßbare Größe der Zellfluoreszenz durch Reflexion an den anorganischen Teilchen verstärkt.

Alternativ kann die Trennschicht aus polymeren Latexkügelchen mit einem Durchmesser vorzugsweise im Bereich von 200 nm bis 5 mm bestehen. Geeignete Polymere sind z.B. Polystyrol, Polyurethan, Butadien Acrylnitril. Die Latexkügelchen können auch mit einem geeigneten Maskierungsfarbstoff angefärbt werden, für den die gleichen Kriterien gelten, wie für den der Lösung zugefügten Absorptionsfarbstoff (s. oben). Eine geeignete Farbstoffklasse sind z.B. ®Resoline.

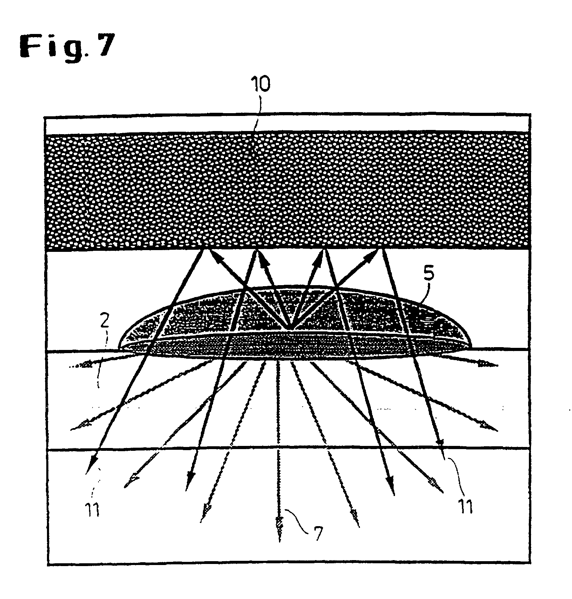

In Lumineszenz-Assays (selbstleuchtende Zellen) besteht grundsätzlich die Anforderung, die spezifische sehr geringe Lichtintensität einer biologischen Zelle mit hoher Empfindlichkeit zu detektieren. Durch Einbringen einer reflektierenden Trennschicht 10 gemäß Fig. 7 kann, analog zur Methode der Unterdrückung der Hintergrundfluoreszenz (gem. Fig. 6), das Lumineszenzsignal der biologischen Zellen verstärkt werden. Hierbei werden Strahlungsanteile 11 des ungerichteten Lumineszenzlichtes in Richtung des Detektors reflektiert und erhöhen so das spezifische Meßsignal.

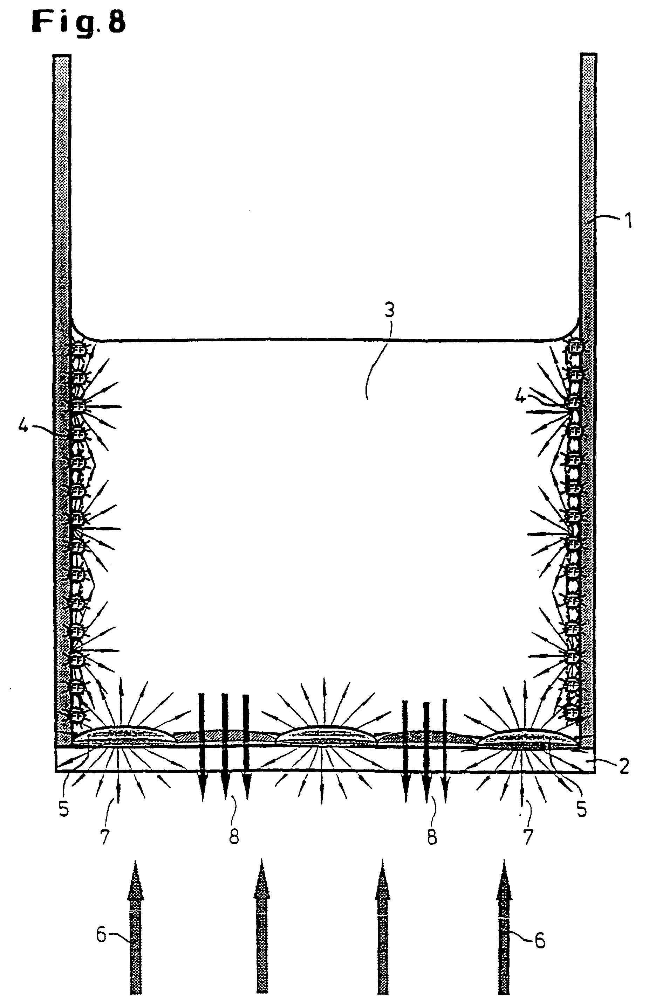

Bei einer Vielzahl anderer fluoreszenter Testverfahren an biologischen Zellen ist es im Gegensatz zu Verteilungsfarbstoffen möglich, den Fluoreszenzfarbstoff nach Anfärbung der Zellen durch Lösungswechsel aus dem Überstand zu entfernen. Der Fluoreszenzfarbstoff FURA2-AM wird z.B. nach Eindringen in die Zelle in den freien Farbstoff gespalten und verliert hierbei seine Zellmembranpermeabilität. Dadurch kommt es zu einer Anreicherung des impermeablen Fluoreszenzfarbstoffes in der Zelle. In diesem Fall kann der fluoreszente Überstand 3 durch eine Fluoreszenzfarbstoff-freie Lösung 3a ausgetauscht werden, ohne die spezifische Zellfluoreszenz zu verändern. Die unspezifische Hintergrundfluoreszenz des Überstandes wird auf diese Weise entfernt. FURA2-AM färbt jedoch Reaktionsgefäße nachhaltig an (Wandfluoreszenz) und erzeugt so ein anderes unspezifisches Fluoreszenzsignal, das der Hintergrund-fluoreszenz von Verteilungsfarbstoffen vergleichbar ist. Dieser Sachverhalt ist in Fig. 8 dargestellt. In diesem Fall geht also die Hintergrundfluoreszenzstrahlung 8 auf die an den Gefäßwänden anhaftenden Fluoreszenzfarbstoffmoleküle 4 zurück. Durch Einbringung von Maskierungsfarbstoffen in den Fluoreszenzfarbstoff-freien Überstand 3a kann auch dieses unspezifische Fluoreszenzsignal vollständig unterdrückt werden.

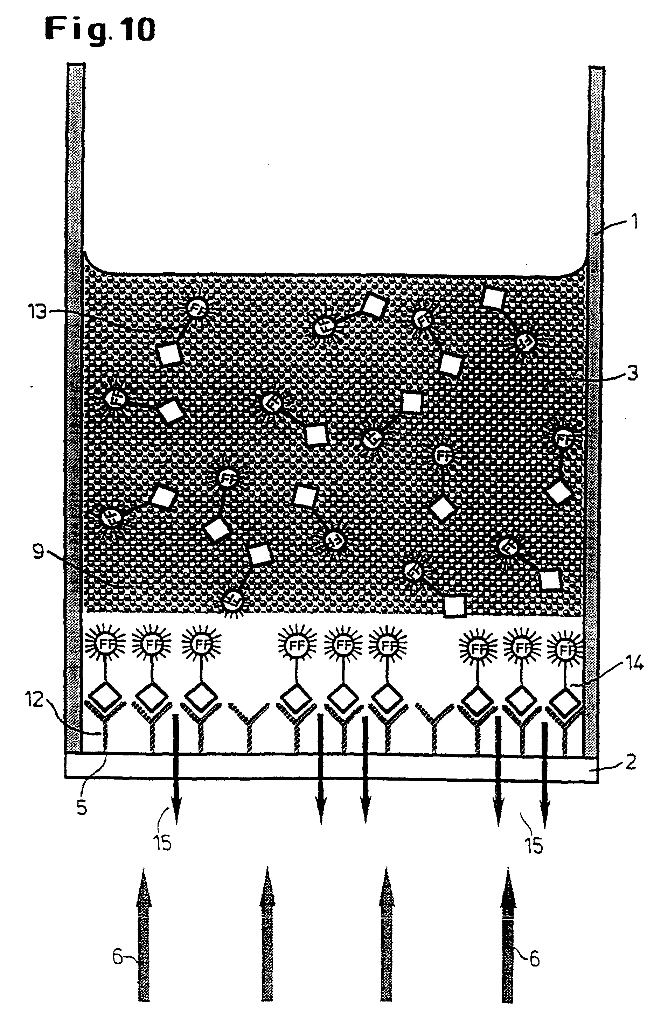

In Fig. 10 ist zusätzlich ein zu Fig. 2 analoges Ausführungsbeispiel dargestellt, bei dem die auf einem transparenten Träger am Boden 2 des Reaktionsgefäßes 1 aufgebrachte biologische Schicht aus Rezeptoren 12 besteht, die mit den im Überstand (Lösung) 3 vorhandenen, Fluoreszenz- oder Lumineszenz-markierten Liganden 13 eine spezifische Bindung eingehen. Die gebundenen Liganden sind hier mit 14 bezeichnet. Das durch den Boden 2 eingestrahlte Primärlicht 6 regt im Falle der nicht maskierten Lösung die Fluoreszenz-markierten Liganden 13 und 14 zur Fluoreszenz an. Im Falle von Lumineszenz-markierten Liganden entfällt das Primärlicht 6. Analog zur Ausführung nach Fig. 2 wird der Lösung 3 wiederum ein Maskierungsfarbstoff beigefügt, der dafür sorgt, daß die von den ungebundenen Liganden 13 ausgehende Fluoreszenz- oder Lumineszenzstrahlung in der Lösung vollständig absorbiert wird. Die am Boden 2 erfaßte Fluoreszenz- oder Lumineszenzstrahlung 15, d.h. der Meßeffekt, geht daher ganz überwiegend auf die an die Rezeptoren 12 gebundenen Liganden 14 zurück und wird nicht durch die Hintergrundstrahlung der ungebundenen Liganden 13 in der Lösung 3 verfälscht. Das Meßsignal ist daher ein direktes Maß für die Stärke der Ligand-Rezeptorbindung. Dabei liegt die Schichtdicke der Rezeptorschicht im nm-Bereich, während die Dimensionen des darüber befindlichen Überstandes in der Größenordnung mehrerer mm liegt.

Gemäß Fig. 6 und 7 kann die für die Lösung durchlässige Trennschicht 10 in ganz analoger Weise bei der Untersuchung von Liganden-Rezeptorbindungen zur Maskierung bzw. Unterdrückung der Hintergrundfluoreszenz bzw. -lumineszenz eingesetzt werden. In diesem Fall wird die störende Hintergrundfluoreszenz bzw. -lumineszenz durch die Trennschicht 10 abgeschirmt und im Falle von lumineszenten Liganden Strahlungsanteile 11 des von gebundenen Liganden stammenden Lumineszenzlichts in Richtung auf den Detektor reflektiert und damit das Nutzsignal verstärkt.

Der in klassischen pharmakologischen Rezeptorbindungsstudien hinsichtlich seiner Bindungsstärke zu bewertende, nicht fluoreszierende bzw. lumineszierende Reaktionspartner wurde hier aus Gründen der Übersichtlichkeit nicht dargestellt.

## Patentansprüche

1. Verfahren zur quantitativen optischen Analyse von fluoreszenzmarkierten biologischen Zellen (5), die in Form einer zusammenhängenden Zellschicht auf einem transparenten Träger am Boden (2) eines Reaktionsgefäßes (1) aufgebracht sind und mit einer den Fluoreszenzfarbstoff (4) enthaltenden Lösung (3) in Kontakt stehen, **dadurch gekennzeichnet, daß** der Lösung (3) zusätzlich zu dem bereits vorhandenen Fluoreszenzfarbstoff (4) ein Maskierungsfarbstoff (9) hinzugegeben wird, welcher das Anregungslicht (6) für den Fluoreszenzfarbstoff (4) und/oder sein Emissionslicht (7) absorbiert und/oder daß auf die Zellschicht eine für die Lösung durchlässige Trennschicht (10) aufgebracht wird, die das Anregungslicht (6) für den Fluoreszenzfarbstoff (4) und/oder sein Emissionslicht (7) absorbiert und/oder reflektiert.

2. Verfahren zur quantitativen optischen Analyse von lumineszenten, biologischen Zellen in Form einer zusammenhängenden, auf dem transparenten Träger befindlichen Zellschicht, **dadurch gekennzeichnet, daß** auf die Zellschicht eine Trennschicht (10) aufgebracht wird, die das Lumineszenzlicht reflektiert.

3. Verfahren zur quantitativen optischen Analyse von Fluoreszenz- oder Lumineszenz-markierten Reaktionspartnern in einem mit einer Lösung (3) gefüllten Reaktionsgefäß (1), in der ein fluoreszierender oder lumineszenter Ligand (13) gelöst ist und die Lösung (3) mit einer für diesen Ligand (13) spezifischen, auf einem transparenten Träger am Boden (2) des Reaktionsgefäß (1) aufgebrachten oder sich darauf absetzenden Rezeptorschicht (12) in Kontakt steht, deren für die Rezeptor-Liganden Bindung charakteristische Fluoreszenz- oder Lumineszenzstrahlung (7,15) durch den transparenten Boden (2) hindurch erfaßt und ausgewertet wird, **dadurch gekennzeichnet, daß** der Lösung (3) ein Maskierungsfarbstoff (9) hinzugefügt und/oder auf die Rezeptorschicht (12) eine für die Lösung (3) durchlässige Trennschicht (10) aufgebracht wird, wobei die optischen Eigenschaften des Maskierungsfarbstoffs (9) und/oder der Trennschicht (10) so gewählt werden, daß das Anregungslicht (6) für den Fluoreszenzfarbstoff (4) des in der Lösung (3) vorhandenen Liganden (13) und/oder sein Fluoreszenzlicht (8) oder sein Lumineszenzlicht von der Lösung (3) oder der Trennschicht (10) absorbiert oder an der Trennschicht (10) reflektiert wird.

4. Verfahren nach Anspruch 1 bis 3, **dadurch gekennzeichnet daß** als Trennschicht (10) eine Schicht aus polymeren Latexkügelchen verwendet wird.

5. Verfahren nach Anspruch 4, **dadurch gekennzeichnet daß** die polymeren Latexkügelchen mit einem Maskierungsfarbstoff gefärbt sind.

6. Verfahren nach Anspruch 1 oder 3, **dadurch gekennzeichnet daß** ein Maskierungsfarbstoff verwendet wird, der eine gute Wasserlöslichkeit und keine zelltoxischen Nebeneffekte aufweist

7. Verfahren nach Anspruch 1 - 2, **dadurch gekennzeichnet daß** bei einem Austausch des einen Fluoreszenzfarbstoff (4) enthaltenden Überstandes (3) durch eine Fluoreszenzfarbstoff-freie Lösung (3a) ein Maskierungsfarbstoff zugegeben wird, welcher die unspezifische, von der angefärbten Reaktionsgefäßwand ausgehende Fluoreszenz unterdrückt.

## Claims

1. Process for the quantitative optical analysis of fluorescently labelled biological cells (5) which are applied to a transparent support at the bottom (2) of a reaction vessel (1) in the form of a coherent cell layer and are in contact with a solution (3) containing the fluorescent dye (4), **characterized in that** the fluorescent dye (4) already present in addition to a masking dye (9) which absorbs the excitation light (6) for the fluorescent dye (4) and/or its emission light (7) is added to the solution (3) and/or **in that** a separating layer (10) which is permeable to the solution and which absorbs and/or reflects the excitation light (6) for the fluorescent dye (4) and/or its emission light (7) is applied to the cell layer.

2. Process for the quantitative optical analysis of luminescent, biological cells in the form of a coherent cell layer situated on the transparent support, **characterized in that** a separating layer (10) which reflects the luminescent light is applied to the cell layer.

3. Process for the quantitative optical analysis of fluorescently or luminescently labelled reaction components in a reaction vessel (1) filled with a solution (3) in which a fluorescent or luminescent ligand (13) is dissolved and the solution (3) is in contact with a receptor layer (12), which is specific for this ligand (13) and is applied to a transparent support at the bottom (2) of the reaction vessel (1) or deposited thereon, whose fluorescent or luminescent radiation (7, 15), which is characteristic of the receptor-ligand binding, is detected and analysed through the transparent bottom (2), **characterized in that** a masking dye (9) is added to the solution (3) and/or a separating layer (10) permeable to the solution (3) is applied to the receptor layer (12), the optical properties of the masking dye (9) and/or of the separating layer (10) being selected such that the excitation light (6) for the fluorescent dye (4) of the ligand (13) present in the solution (3) and/or its fluorescent light (8) or its luminescent light is absorbed by the solution (3) or the separating layer (10) or reflected at the separating layer (10).

4. Process according to Claims 1 to 3, **characterized in that** the separating layer (10) used is a layer of polymeric latex beads.

5. Process according to Claim 4, **characterized in that** the polymeric latex beads are dyed with a masking dye.

6. Process according to Claim 1 or 3, **characterized in that** a masking dye is used which possesses good water solubility and has no cytotoxic side effects.

7. Process according to Claim 1 - 2, **characterized in that** in the case of a_ replacement of the supernatant (3) containing a fluorescent dye (4) by a fluorescent dye-free solution (3a) a masking dye is added which suppresses the non-specific fluorescence emitted from the stained reaction vessel wall.

## Revendications

1. Méthode d'analyse optique quantitative de cellules biologiques (5) marquées par la fluorescence, qui sont déposées sous forme d'une couche cellulaire cohérente sur un support transparent au fond (2) d'un récipient de réaction (1) et qui sont en contact avec une solution (3) contenant le colorant fluorescent (4), **caractérisée en ce qu'**on ajoute à la solution (3) en plus du colorant fluorescent (4) déjà présent un colorant de masquage (9) qui absorbe la lumière d'excitation (6) pour le colorant fluorescent (4) et/ou sa lumière d'émission (7) et/ou **en ce qu'**une couche de séparation (10) perméable à la solution, qui absorbe et/ou réfléchit la lumière d'excitation (6) pour le colorant fluorescent (4) et/ou sa lumière d'émission (7), est disposée sur la couche cellulaire.

2. Méthode d'analyse optique quantitative de cellules biologiques luminescentes sous forme d'une couche cellulaire cohérente se trouvant sur le support transparent, **caractérisée en ce qu'**une couche de séparation (10) qui réfléchit la lumière produite par luminescence est disposée sur la couche cellulaire.

3. Méthode d'analyse optique quantitative de partenaires réactionnels marqués par la fluorescence ou la luminescence dans un récipient de réaction (1) rempli d'une solution (3) dans laquelle est dissous un ligand fluorescent ou luminescent (13) et la solution (3) est en contact avec une couche réceptrice (12) spécifique de ce ligand (13), disposée ou se déposant sur un support transparent au fond (2) du récipient de réaction (1), dont le rayonnement fluorescent ou luminescent (7, 15) caractéristique de la liaison récepteur-ligand est perçu à travers le fond transparent (2) et est évalué, **caractérisée en ce qu'**un colorant de masquage (9) est ajouté à la solution (3) et/ou une couche de séparation (10) perméable à la solution (3) est disposée sur la couche réceptrice (12), les propriétés optiques du colorant de masquage (9) et/ou de la couche de séparation (10) étant choisies de manière que la lumière d'excitation (6) pour le colorant fluorescent (4) du ligand (13) présent dans la solution (3) et/ou sa lumière fluorescente (8) ou sa lumière luminescente soient absorbées par la solution (3) ou la couche de séparation (10) ou soient réfléchies sur la couche de séparation (10).

4. Méthode suivant les revendications 1 à 3, **caractérisée en ce qu'**on utilise comme couche de séparation (10) une couche formée de petites billes de latex polymère.

5. Méthode suivant la revendication 4, **caractérisée en ce que** les petites billes de latex polymère sont colorées avec un colorant de masquage.

6. Méthode suivant la revendication 1 ou 3, **caractérisée en ce qu'**on utilise un colorant de masquage qui présente une bonne solubilité dans l'eau et qui ne produit pas d'effets secondaires toxiques pour les cellules.

7. Méthode suivant les revendications 1 et 2, **caractérisée en ce qu'**un colorant de masquage qui supprime la fluorescence non spécifique provenant de la paroi teintée du récipient de réaction est ajouté en cas de remplacement du liquide surnageant (3) contenant un colorant fluorescent (4) par une solution (3a) ne contenant pas de colorant fluorescent.
